# EUROPEAN PATENT APPLICATION

(11) **EP 2 599 786 A1**
(43) Date of publication of application: **05.06.2013**
(21) Application number: 11812398.3
(22) Date of filing: 22.07.2011
(51) Int. Cl.: C07K 1/02

(54) **METHOD FOR PRODUCING PEPTIDE**

(30) Priority: 26.07.2010 JP 2010167212
(71) Applicant: Ajinomoto Co., Inc., Tokyo 104-8315 (JP)
(72) Inventor: FURUKAWA Shinya, Kawasaki-shi Kanagawa 210-8681 (JP); HASEGAWA Kazuhiro, Kawasaki-shi Kanagawa 210-8681 (JP); FUKE Ichiro, Kawasaki-shi Kanagawa 210-8681 (JP)
(74) Representative: Nicholls, Kathryn Margaret
(86) International application number: PCT/JP2011/066713
(87) International publication number: WO 2012/014808

(57) **Abstract**

Provided is a method for producing a peptide, which comprises the steps of allowing (A) a first amino acid or peptide, which is converted into its ionic liquid form, (B) a second amino acid or peptide and (C) a peptide hydrolase to simultaneously exist at a single reaction system, wherein the first amino acid or peptide, which is converted into its ionic liquid form, is used as both a reaction solvent and a reaction starting material; and forming a peptide bond between the first amino acid or peptide and the second amino acid or peptide. It is possible to synthesize a peptide at a high concentration and at a high yield by using said method and said method is excellent for producing peptides at an industrial scale.

## Description

### Technical Field

The present invention relates to a method for producing a peptide in a high yield, utilizing an ionic liquid. In particular, the present invention relates to a method for producing a peptide in an industrial scale.

### Background Art

As it would be anticipated that peptides have a great demand as active components of pharmaceutical products, they have conventionally been produced according to a variety of methods. Then there has recently been proposed a method for producing a peptide utilizing an ionic liquid and such a method has accordingly become of major interest. For instance, Patent Document 1 discloses a method for using an ionic liquid to combine with oligo-peptide, an oligo-saccharide or an oligo-nucleotide for improving the solubility in an organic solvent, and for utilizing as a protective group. However, in this method, a protective group and a condensation agent are required in each polymerization reaction. In Non-Patent Document 1, the reaction: Z-Asp + PM → Z-APM is conducted in an ionic liquid (BP6 [1-butyl-3-methyl-imidazolium hexafluoro phosphate)] utilizing an enzyme (Thermolysin). Thus it is established that an enzymatic reaction can be carried out even in an ionic liquid. The yield of this reaction is high on the order of 90%, but the reaction should be conducted at considerably low concentrations of reactants. For this reason, the reaction disclosed in this article is considered to be an enzymatic reaction-in an organic solvent in which a solvent is simply substituted. In addition, Patent Document 2 discloses the synthesis of a peptide in an ionic liquid (4-methyl-N-butyl-pyridinium tetrafluoro borate). The synthesis herein is also considered to be an enzymatic reaction-in an organic solvent in which a solvent is simply substituted, as well as Non-Patent Document 1. Moreover, in this synthesis method, the reaction is carried out at a considerably low concentration on the order of 20mM and the reaction requires the use of a protective group.
Non-Patent Document 2 establishes that an amino acid can be converted into its ionic liquid form by combining the amino acid with a residual group of an ionic liquid through an ionic bond. Although the document does not discuss about its application, a use for an electrolyte of a fuel cell has initially been investigated. Non-Patent Document 3 describes a review of the synthesis of polypeptides, oligosaccharides or other organic substances using an ionic liquid and the gist thereof describes that a substrate-ionic liquid is used as an intermediate for such a synthesis. However, there is no specific data. Non-Patent Document 4 discloses an introductory review of an ionic liquid combined with an amino acid and it also refers to the applications thereof as a solvent or a catalyst in the near future, although there is not disclosed therein any specific data at all.
However, the methods for synthesizing a peptide utilizing an ionic liquid, which have been proposed until now, provide the peptide in a low yield and neither of them is a method for producing a peptide in an industrial scale.

### Prior Art Documents

### Patent Documents:

Patent Document 1: JP-T-2008-537733
Patent Document 2: JP-A-2008-301829

### Non-Patent Documents:

Non-Patent Document 1: Biotechnol. Prog., 2000, 16: 1129-1131
Non-Patent Document 2: Acc. Chem. Res., 2007, 40: 1122-1129
Non-Patent Document 3: "Advance in Liquid-Phase Organic Synthesis Using Functional Ionic Liquid as Supports", Nanjing University of Technology, HU Yi, LI Heng, HUANG He, WEI Ping, Issued on March, 2007
Non-Patent Document 4: "Progress on Amino Acid-Ionic Liquid", Liaoning University, WU Yang, ZHANG Tian-tian, SONG Xi-ming, Issued on March, 2008

### Disclosure of the Invention

An object of the present invention is to provide a method for producing a peptide at a high concentration and in a high yield utilizing an ionic liquid and, in particular, to a method for producing a peptide in an industrial scale.

The present invention has been completed by finding that the aforementioned problems can efficiently be solved by using a first amino acid or peptide, which is converted into its ionic liquid form, as a reaction starting material and a reaction solvent, and reacting the first amino acid or peptide, which is converted into its ionic liquid form, with a second amino acid or peptide at a reaction system wherein these two components and (C) a peptide hydrolase are allowed to simultaneously exist.
More specifically, the present invention provides a method for producing a peptide, which comprises the steps of allowing (A) a first amino acid or peptide, which is converted into its ionic liquid form,, (B) a second amino acid or peptide and (C) a peptide hydrolase to simultaneously exist at a single reaction system, wherein the first amino acid or peptide, which is converted into its ionic liquid form, is used as both a reaction solvent and a reaction starting material; and
forming a peptide bond between the first amino acid or peptide and the second amino acid or peptide.

Thus the present invention permits the synthesis of a peptide at a high concentration and in a high yield. In the method of this invention, a peptide hydrolase controls the amino acids-sequence of a final reaction product, i.e. the peptide, by its position-specificity.

### Mode for Carrying Out the Invention

First of all, (A) a first amino acid or peptide, which is converted into its ionic liquid form through the formation of an ionic bond, is used as a reaction solvent and a reaction starting material, in the present invention. In this respect, the first amino acid or peptide is converted into its ionic liquid form through the formation of an ionic bond of corresponding amino acid or peptide and a cation derived from a quaternary hetero atom-containing compound such as one selected from the group consisting of a quaternary phosphonium salt, a quaternary ammonium salt, an imidazolium salt, a pyridinium salt, a pyrrolidinium salt and a piperidinium salt. More specifically, a first amino acid or peptide isconverted into its ionic liquid form through an ionic bond of the corresponding amino acid or peptide and at least one cation selected from the group consisting of alkyl phosphonium ions, alkyl imidazolium ions, alkyl ammonium ions, alkyl pyridinium ions, alkyl pyrrolidinium ions and alkyl piperidinium ions. The number of carbon atoms of each of the alkyl groups included in the foregoing alkyl phosphonium ions or the like preferably falls within the range of from 1 to 12, more preferably 1 to 6 and most preferably 1 to 4. In the instance where such a quaternary cation contains a plurality of alkyl groups, the alkyl groups may be the same or different from one another, but they are preferably identical to one another. More specifically, preferred examples of the foregoing quaternary cations include a tetrabutyl phosphonium ion, a tetraethyl phosphonium ion, a tetramethyl quaternary ammonium ion, a tetraethyl quaternary ammonium ion, a tetrabutyl quaternary ammonium ion, a hexyl triethyl quaternary ammonium ion, a 1-ethyl-3-methyl-imidazolium ion, a 1,3-dimethyl-imidazolium ion, a 1-butyl-3-methyl-imidazolium ion, a 1-butyl-3-methyl-pyridinium ion, a 1-butyl-pyridinium ion and a 1-methyl- 1-butyl-pyrrolidinium ion. These quaternary cations can easily be available in the form of, for instance, hydrochlorides, hydrobromides and hydroxides thereof from, for instance, Tokyo Chemical Industries, Co., Ltd., Hokko Chemical Co., Ltd., and Toyo Synthetic Chemical Co., Ltd.
In this connection, the term "ionic liquid" in this specification does not mean any molten or fused salt, but means a salt constituted by an ion, which can be fused at a low temperature of not higher than 100°C. Accordingly, water never falls within the purview of the ionic liquid to be used herein.

In the present invention, the foregoing first amino acid or peptide may be, for instance, essential amino acids such as proline (Pro), tyrosine (Tyr), phenylalanine (Phe), leucine (Leu), glycine (Gly), methionine (Met), serine (Ser), alanine (Ala), aspartic acid (Asp), glutamine (Gln), glutamic acid (Glu), histidine (His), lysine (Lys) and valine (Val) and analogues of these amino acids as well as oligomers and polymerized products (polymers) thereof. Among them, aliphatic amino acids and oligomers containing such amino acids are preferably used as the constituents of the oligomers. Moreover, the first amino acids and peptides may be protected at their amino groups or carboxyl groups. In particular, the amino group present in each of these amino acids or peptides may be protected with a group for protecting an amino group such as a formyl group, a benzyloxy-carbonyl group or a butoxycarbonyl group.
In the present invention, the foregoing quaternary hetero atom-containing compound and the first amino acid or peptide can be mixed together in approximately equivalent molar amounts; and the water in the resulting mixture can then be evaporated by heating the mixture (preferably at a temperature ranging from 40 to 70°C) under ordinary pressure or under reduced pressure (preferably at a pressure ranging from about 2.7 kPa to about 20 kPa (20 to 150 mmHg)) to subject the mixture to a dehydration condensation reaction and to thereby form the corresponding first amino acid or peptide, which is converted into its ionic liquid form. In this connection, the reaction is conducted under reduced pressure for avoiding any decomposition of the substrate, in Examples as described later.
In the present invention, preferably used as (A) the first amino acid or peptide, which is converted into its ionic liquid form, is a carboxylate, i.e. the first amino acid or peptide is ionically bonded to the foregoing quaternary hetero atom-containing compound through the carboxyl group present in the first amino acid or peptide.
In this respect, the disclosure of Non-Patent Document 2 (Acc. Chem. Res., 2007, 40: 1122-1129, which relates to quaternary hetero atom-containing compounds, amino acids and peptides as well as amino acids or peptides converted into their ionic liquid forms) should be construed as being substantially disclosed in the original specification of the present patent application.

In the present invention, the second component, which is to be reacted with (A) the foregoing component, are (B) second amino acids or peptides. In this respect, amino acid esters or peptide esters may be used as the second component (B). The amino acids or peptides used herein may be, for instance, essential amino acids such as proline (Pro), tyrosine (Tyr), phenylalanine (Phe), leucine (Leu), glycine (Gly), methionine (Met), serine (Ser), alanine (Ala), aspartic acid (Asp), glutamine (Gln), glutamic acid (Glu), histidine (His), lysine (Lys) and valine (Val) and analogues of these amino acids as well as oligomers and polymerized products (polymers) thereof, whose carboxyl group has been esterified with, for instance, alkyl groups. In this connection, the alkyl groups may preferably have 1 to 12 carbon atoms, more preferably 1 to 6 carbon atoms and particularly preferably 1 to 4 carbon atoms. These esters may be used alone or in any combination of at least two of them. In addition, a component (B) may be a form of an acid-addition salt derived from an inorganic acid, for instance, hydrochloride.
Among these substances, amino acids having an aromatic ring or a hetero ring within the molecule and oligomers containing the same are preferably used as their constituents, and particularly, phenylalanine (Phe) methyl ester and the like are preferably used herein.

In the present invention it is preferred that the second amino acid or peptide is not protected at its amino group, but the second amino acids or peptides may be protected at its amino group.
In the present invention, (B) the second amino acids or peptides may be converted into their ionic liquid forms. In this case, the same method used for the conversion of the foregoing component (A) can be used for the preparation of the component (B), while using the same quaternary hetero atom-containing compounds described above in connection with the conversion of an amino acid into its ionic liquid form. In this case, it is preferred that (D) water is present in the reaction system. The amount thereof is preferably not more than 50% by mass and particularly preferably 2 to 20% by mass on the basis of the total mass of the reaction system.

The present invention is characterized in that (A) the aforementioned first amino acid or peptide, which is converted into its ionic liquid form through the formation of an ionic bond, is used as a reaction solvent and a reaction starting material, and that the component (A) is then reacted with (B) the foregoing second amino acid or peptide. More specifically, there is substantially no reaction solvent other than the component (A), i.e. the first amino acid or peptide, which is converted into its ionic liquid form. In other words, the reaction proceeds in such a condition that the component (B), which is the second amino acid or peptide, is dissolved in the component (A). For this reason, the component (A) is used in an amount of not less than the equimolar amount relative to the component (B), preferably at a molar ratio, i.e. the molar amount of the first amino acid: that of the second amino acid ranging from 20:1 to 1:1, and more preferably 10:1 to 2:1. However, in the instance where the component (B) has a poor solubility in the component (A), undissolved state of (B) the second amino acid or peptide would gradually be dissolved in the component (A) along with the progress of the reaction of the component (A) with the component (B). Therefore, the ratio between the amounts of the components (A) and (B) may be determined depending on the characteristic properties of these components (A) and (B).
According to the present invention, the first amino acid or peptide, which is converted into its ionic liquid form, may be used as a reaction solvent and a reaction starting material and may be reacted with the esters of second amino acid or peptide in such a condition that the first amino acid or peptide is present in excess. This accordingly permits the selective production of an intended peptide without introducing any protective group into the second amino acid.
In the present invention, it would be preferred to form a peptide bond between the carboxyl group present in the first amino acid or peptide and the amino group present in the second amino acid or peptide according to the foregoing reaction.

The present invention is characterized in that the component (A) and the component (B) are reacted in the presence of (C) a peptide hydrolase to form a peptide bond between the first amino acid or peptide and the second amino acid or peptide. When conducting this reaction according to the present invention, it is preferred that water is present in the reaction system in an amount of not more than 50% by mass and, in particular, 5 to 20% by mass relative to the total mass of the reaction system.
In this connection, the peptide hydrolase usable herein is preferably at least one member selected from the group consisting of proteases, peptidases and hydrolases. The use of thermolysin is particularly preferred in the present invention. Such an enzyme can easily be available from Sigma-Aldrich Corporation.
Such a peptide hydrolase (C) is preferably present in the reaction system in an amount ranging from 1 to 4% by mass, based on the total mass of the reaction system.
In the method of this invention, a peptide hydrolase is used and therefore, the sequence of the amino acids constituting the peptide as a final reaction product can easily be controlled, while making the most use of the position- or regio-specificity of the enzyme.
The reaction system according to the present invention contains a substantial quantity of water and therefore, it is preferred to control the pH value of the reaction system to a level ranging from 4 to 10.5.

In the present invention, the reaction of (A) the first amino acid or peptide, which is converted into its ionic liquid form, with (B) the second amino acid or peptide is conducted by blending these two reagents together and then maintaining the reaction system at a temperature ranging from 0 to 100°C, preferably room temperature (20°C) to 70°C and more preferably 30°C to 40°C. The completion of the reaction is preferably confirmed by detecting the end point of the peptide-forming reaction according to the HPLC technique, the final reaction product is preferably isolated by means of, for instance, a method utilizing a resin, a method utilizing an organic solvent and a method utilizing the crystallization technique and the identification of the reaction product is desirably carried out according to the HPLC technique.
In the instance where first and second amino acids or peptides, whose amino groups or carboxyl groups are protected, as the reaction starting materials, these protective groups may be eliminated or removed (deblocking) according to the usual technique such as a catalytic reduction technique.
The peptides (oligopeptides or polypeptides) prepared by the synthetic method according to the present invention can be widely used as effective components for foods including, for instance, functional foods and seasonings, nutrient compositions such as infusions and livestock feeds; active components for pharmaceutical products; or effective components for a variety of chemical reagents.
Then the present invention will be described in more detail with reference to the following working Examples.

### Examples

### Example 1: Effect on Dipeptide-Forming Enzyme Reaction, of Using Amino Acid; Benzyloxy-carbonyl Aspartic Acid (Z-Asp) which is converted into its ionic liquid form, Serving as Reaction Starting Material and as Reaction Solvent to Increase the Concentration of the Amino Acid.

Equimolar amounts of commercially available Z-Asp and a 40% by mass solution of tetrabutyl phosphonium hydroxide (hereunder referred to as "TBP-OH") (mixing ratio: Z-Asp/TBO-OH = 1:1) were blended together (50 g in total), the resulting mixture was then stirred in a water bath warmed at 60°C, the pressure of the reaction system was about 6.7kPa (50mmHg) to make the water evaporate from the reaction system and to thereby allow a dehydration condensation reaction of these components to take place. The obtained benzyloxy-carbonyl aspartic acid-tetrabutyl phosphonium (hereunder referred to as "Z-Asp-TBP") was found to be a colorless and transparent liquid.
Sulfuric acid and methanol were added to phenylalanine (hereunder referred to as "Phe"), in amounts of 1.2 times and 5 times the amount of the phenylalanine, respectively and then the resulting mixture was stirred while the distillable or volatile component present in the mixture was refluxed in a water bath at 70°C for 30 minutes. Thereafter the preset temperature of the water bath was raised up to 120°C to allow the volatile component to discharge from the reaction system till the temperature of the reaction vessel arrived at a level of 90°C. Once the temperature of the reaction liquid present in the reaction vessel reached 90°C, the addition of supplemental methanol was initiated and the temperature of the reaction liquid was maintained at 90°C, while the volatile component was distilled off from the reaction liquid. The reaction liquid was recovered 6.5 hours after the initiation of the addition of the supplemental methanol. In this way, there was ultimately prepared phenylalanine methyl ester-monomethyl sulfate (hereunder referred to as "PM-MHS").

PM-MHS was completely dissolved in the foregoing Z-Asp-TBP in the form of an ionic liquid which was prepared according to the foregoing procedures, in such a manner that the concentration of the PM-MHS was controlled to a level of 100mmol/L in the Z-Asp-TBP. Then the resulting solution was warmed to 37°C in a water bath and the pH value thereof was adjusted to a level of 6.0 using a 25g/dL Na₂CO₃ aqueous solution. Then thermolysin as an enzyme was added to the solution in an amount of 10mg/mL relative to the amount of the Z-Asp-TBP to initiate the enzyme reaction (the amount of water relative to the total mass of the reaction system: 10% by mass). The enzyme reaction was finished or terminated 48 hours after the initiation of the reaction, and the samples collected at certain intervals of time during the reaction were analyzed according to the HPLC technique to confirm the formation of the intended Z-aspartic acid phenylalanine methyl ester (Z-APM). The results obtained are summarized in the following Table 1.
The amino acids, Z-Asp and Phe, used in the above procedures are commercially available and the thermolysin used in this Example was one purchased from Sigma Company. In addition, TBP-OH was purchased from Hokko Chemical Industry Co. , Ltd.

**Table 1:**

| | Added Amount of Thermolysin | |
|---|---|---|
| | Not Added | 10mg/mL |
| Yield of Z-APM (%) | 0.0 | 18.8 |

### Example 2: Effect of Concentration of PM-MHS on Z-APM-Forming Enzyme Reaction:

PM-MHS was completely dissolved in the Z-Asp-TBP disclosed in Example 1 and prepared by converting Z-Asp into its ionic liquid form such that the concentration of the PM-MHS was equal to 100 or 1,000mmol/L in the Z-Asp-TBP, then the resulting solution was warmed at 37°C in a water bath and the pH value thereof was adjusted to a level of 6.0 using a 25g/dL Na₂CO₃ solution. Then thermolysin as an enzyme was added to the solution in an amount of 10mg per unit volume (1mL) of Z-Asp-TBP to initiate the desired enzyme reaction (the amount of water relative to the total mass of the reaction system: 10% by mass) . The enzyme reaction was finished 48 hours after the initiation of the enzyme reaction and the samples collected at certain intervals of time during the reaction were analyzed according to the HPLC technique to confirm the formation of the intended Z-APM (benzyloxy-carbonyl aspartic acid phenylalanine methyl ester). The results obtained are summarized in the following Table 2.

**Table 2:**

| | Concentration of PM | |
|---|---|---|
| | 100mmol/L | 1,000mmol/L |
| Yield of Z-APM (%) | 18.8 | 45.8 |

### Example 3: Effect of Concentration of Thermolysin on Z-APM-Forming Enzyme Reaction:

There was completely dissolved PM-MHS in the Z-Asp-TBP disclosed in Example 1 and prepared by converting Z-Asp into its ionic liquid form such that the concentration of the former was equal to 1,000mmol/L in the Z-Asp-TBP, then the resulting solution was warmed at 37°C in a water bath and the pH value thereof was adjusted to a level of 6.0 using a 25g/dL Na₂CO₃ solution. Then thermolysin as an enzyme was added to the solution in an amount of 10mg or 40mg per unit volume (1mL) of Z-Asp-TBP to initiate the desired enzyme reaction (the amount of water relative to the total mass of the reaction system: 10% by mass). The enzyme reaction was finished 48 hours after the initiation of the enzyme reaction and the samples collected at certain intervals of time during the reaction were analyzed according to the HPLC technique to confirm the formation of the intended Z-APM. The results obtained are summarized in the following Table 3.

**Table 3:**

| | Concentration of Thermolysin | |
|---|---|---|
| | 10mg/mL | 40mg/mL |
| Yield of Z-APM (%) | 45.8 | 77.6 |

### Example 4: Effect on Formyl Aspartic Acid Phenylalanine Methyl Ester (F-APM)-Forming Enzyme Reaction, of Using Amino Acid Amino Acid; Formyl Aspartic Acid (F-Asp) which is converted into its ionic liquid form, Serving as Reaction Starting Material and Reaction Solvent to Increase the Concentration of the Amino Acid.

Anhydrous formyl aspartic acid (hereunder referred to as "F-Asp=O") prepared according to the method disclosed in J P-B-1980-26133, was suspended in acetic acid methyl ester whose amount was 10 times that of the F-Asp=O, further water was added to the mixture in an amount of 1.5 times that of the F-Asp=O and the resulting mixture was stirred at room temperature for 6 hours. Then the crystals formed and separated out of the mixture were recovered through filtration to give the intended F-Asp.
Equimolar amounts of the F-Asp prepared according to the foregoing method and a 40% TBP-OH solution purchased from the market (Z-Asp: TBP-OH =1:1) were blended, the resulting mixture was stirred in a water bath warmed at 60°C, while reducing the pressure of the reaction system to about 6. 7kPa (50mmHg) to make the water evaporate from the reaction system and to thereby allow a dehydration condensation reaction of these components to take place. It was found that the formyl aspartic acid tetrabutyl phosphonium (F-Asp-TBP) prepared was a colorless and transparent liquid.
The F-Asp-TBP prepared above by converting F-Asp into its ionic liquid form and PM-MHS were uniformly blended such that the concentration of the PM-MHS was set at 1,000mmol/L in the F-Asp-TBP, the resulting mixture was warmed at 37°C in a water bath and then the pH value of the mixture was controlled to a level of 6.0 using a 25g/dL Na₂CO₃ solution. Then thermolysin was added to the mixture in an amount of 40mg per 1mL of F-Asp-TBP to permit the initiation of the enzyme reaction. The enzyme reaction was finished 48 hours after the initiation of the enzyme reaction and the samples collected at certain intervals of time during the reaction were analyzed according to the HPLC technique to confirm the formation of the intended formyl aspartic acid phenylalanine methyl ester (hereunder referred to as "F-AMP"). The results obtained are summarized in the following Table 4.

**Table 4:**

| | Concentration of Thermolysin | |
|---|---|---|
| | Not Added | 40mg/mL |
| Yield of F-APM (%) | 0.0 | 4.6 |

### Example 5: Effect of Ionic Liquid Species Used in Conversion of Amino Acid into Its ionic Liquid on Z-AMP-Forming Enzyme Reaction:

Equimolar amounts of Z-Asp and a 40% tetraethyl ammonium hydroxide solution (hereunder referred to as "TEA-OH") (molar ratio: Z-Asp: TEA-OH =1:1) were blended, the resulting mixture was stirred in a water bath warmed at 60°C, while reducing the pressure of the reaction system to about 6.7kPa (50mmHg) to make the water evaporate from the reaction system and to thereby allow a dehydration condensation reaction of these components to take place. It was found that the benzyloxy- carbonyl aspartic acid tetraethyl ammonium (hereunder referred to as "Z-Asp-TEA") prepared was a colorless and transparent liquid.
PM-MHS was completely dissolved in the Z-Asp-TEA prepared by converting Z-Asp into its ionic liquid form according to the foregoing method, such that the concentration of the PM-MHS was equal to 1,000mmol/L in the Z-Asp-TBP, then the resulting solution was warmed at 37°C in a water bath and the pH value thereof was adjusted to a level of 6.0 using a 25g/dL Na₂CO₃ solution. Then thermolysin as an enzyme was added to the solution in an amount of 40mg per unit volume (1mL) of Z-Asp-TBP to initiate the intended enzyme reaction. The enzyme reaction was finished 48 hours after the initiation of the enzyme reaction and the samples collected at certain intervals of time during the reaction were analyzed according to the HPLC technique to confirm the formation of the intended Z-APM. The results obtained are summarized in the following Table 5.
In this respect, the reagent TEA-OH used in this Example was purchased from Toyo Synthetic Chemical Co., Ltd.

**Table 5:**

| | Concentration of Thermolysin | |
|---|---|---|
| | Not Added | 40mg/mL |
| Yield of Z-APM (%) | 0.0 | 61.4 |

## Claims

1. A method for producing a peptide, which comprises the steps of allowing (A) a first amino acid or peptide, which is converted into its ionic liquid form, (B) a second amino acid or peptide and (C) a peptide hydrolase to simultaneously exist at a single reaction system, wherein the first amino acid or peptide, which is converted into its ionic liquid form, is used as both a reaction solvent and a reaction starting material; and
forming a peptide bond between the first amino acid or peptide and the second amino acid or peptide.

2. The method as set forth in claim 1, wherein the second amino acid or peptide (B) is converted into its ionic liquid form.

3. The method as set forth in claim 2, wherein water is present in the reaction system.

4. The method as set forth in any one of claims 1 to 3, wherein the first amino acid or peptide of (A) is protected at its amino group or carboxyl group.

5. The method as set forth in any one of claims 1 to 3, wherein (A) the first amino acid or peptide, which is converted into its ionic liquid form, is a carboxylate.

6. The method as set forth in claim 5, wherein (A) the first amino acid or peptide, which is converted into its ionic liquid form, is protected at its amino group.

7. The method as set forth in claim 1, wherein (A) the first amino acid or peptide is converted into its ionic liquid form through the formation of an ionic bond of at least one amino acid or peptide and at least one cation selected from the group consisting of alkyl phosphonium ions, alkyl imidazolium ions, alkyl ammonium ions, alkyl pyridinium ions, alkylpyrrolidinium ions and alkyl piperidinium ions.

8. The method as set forth in any one of claims 1 to 7, wherein the peptide hydrolase (C) is at least one member selected from the group consisting of proteases, peptidases and hydrolases.

9. The method as set forth in claim 8, wherein the peptide hydrolase (C) is thermolysin.

10. The method as set forth in any one of claims 1 to 9, wherein the content of water in the reaction system is not more than 50% by mass.

11. The method as set forth in any one of claims 1 to 10, wherein the peptide bond is formed at a temperature ranging from 0 to 100°C.

12. The method as set forth in claim 11, the peptide bond is formed at a temperature ranging from room temperature to 70°C.
